(19) Europäisches Patentamt / European Patent Office / Office européen des brevets

(11) **EP 1 858 352 B1**

(12) **EUROPEAN PATENT SPECIFICATION**

(45) Date of publication and mention of the grant of the patent:
**05.06.2013 Bulletin 2013/23**

(21) Application number: **06716901.1**

(22) Date of filing: **17.02.2006**

(51) Int Cl.:
*A23L 1/30* (2006.01)　　*A23L 2/40* (2006.01)

(86) International application number:
**PCT/SE2006/000211**

(87) International publication number:
**WO 2006/088418 (24.08.2006 Gazette 2006/34)**

(54) **A COMPOSITION COMPRISING A POWDER CONTAINING MICROENCAPSULATED POLYUNSATURATED LONG-CHAIN ESTERIFIED FATTY ACIDS DISTRIBUTED IN AN EFFERVESCENT BASE**

ZUSAMMENSETZUNG UMFASSEND EIN PULVER ENTHALTEND MIKROVERKAPSELTE MEHRFACH UNGESÄTTIGTE LANGKETTIGE VERESTERTE FETTSÄUREN, DIE IN EINEM BRAUSEGRUNDSTOFF VERTEILT SIND

COMPOSITION COMPRENANT UNE POUDRE CONTENANT DES ACIDES GRAS ESTERIFIES A LONGUE CHAINE POLYINSATURES ET MICROENCAPSULES DISTRIBUEE DANS UNE BASE EFFERVESCENTE

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.02.2005 SE 0500380**
**18.02.2005 US 654736 P**

(43) Date of publication of application:
**28.11.2007 Bulletin 2007/48**

(73) Proprietor: **Delante Health AS**
**1357 Bekkestua (NO)**

(72) Inventors:
• **WAHREN, Robert**
**S-236 33 Höllviken (SE)**

• **SKJAEVESTAD, Björn**
**NO-1357 Bekkestua (NO)**

(74) Representative: **Holmberg, Nils Anders Patrik**
**BRANN AB**
**P.O. Box 12246**
**102 26 Stockholm (SE)**

(56) References cited:
| EP-A1- 0 266 323 | WO-A1-91/04757 |
| WO-A1-02/074308 | WO-A2-2006/067647 |
| US-A- 4 725 427 | US-A- 4 895 725 |
| US-A- 5 560 928 | US-A- 5 560 928 |
| US-A1- 2002 032 234 | US-A1- 2003 170 301 |

**Description**

FIELD OF INVENTION

The invention relates to a composition being substantially free from water comprising a powder containing microencapsulated polyunsaturated long-chain esterified fatty acids (PUFA), wherein said PUFA content of said powder is from 5 to 50 %(w/w), said powder being homogenously distributed in an effervescent base, wherein said effervescent base is from 20 to 75 % (w/w) of said composition. The invention also relates to a process for the production of said composition as well as tablets, granules and powders comprising said composition and does unites as well as containers comprising said composition, tablet, granules or powders and the use of said products.

BACKGROUND OF INVENTION

Today, there are several techniques used in the preparation of oil-based dietary formulations, such as soft gel capsules and emulsions containing oil-based biological materials. For example, fish oil is the richest dietary source of long-chain omega-3 polyunsaturated fatty acids (PUFA). Long-chained oils containing polyunsaturated fatty acids (PUFA) in pure form or as an aqueous emulsion are very susceptible to oxidation giving them rapidly an unpleasant taste. Stabilisation of omega-3, omega-6 PUFA or other oil-based materials against oxidation by the addition of substances with antioxidative effects is an important task in food industry. Another example is where the oil-based material is administered in airtight gelatine capsules. Use of such capsules has both reduced the oxidation of omega-3 PUFA as well as other oils. However, many consumers such as children and senior people have problems in swallowing large capsules and swallowing of the capsules may give rise to burping and "fish breath". Another way to stabilise these oil-based materials is by microencapsulation, eg. US 4,895,725 disclosing a microencapsulation of fish oil using cellulose as a coating agent. During recent years microencapsulated oils containing polyunsaturated fatty acids have been developed for formulation in food, like bread, but intake of nutritionally relevant components like fish oil is hard to monitor when taken as an integrated part of food. ISSFAL (International Society for the Study of Fatty Acids and Lipids) recognized that there may be a healthy upper limit of the intake of linoleic acid.

EP 266 323 discloses compositions comprising oil-powder mixtures containing 40 to 75 % (w/w) oil and an acid/bicarbonate mixture, e.g., a citric acid/sodium bicarbonate mixture makes the tablet effervescent upon addition of water.

Effervescent tablets and granules has so far been utilised as an oral delivery system in the pharmaceutical industry for decades. Effervescent technology has traditionally been used for delivery of antacid and pain relief medicine like Alka-Seltzer. For patients who have problems with swallowing of normal tablets, effervescent tablets have been a favoured dosage form. Nutritional supplements of vitamins, minerals and amino acids is also an area were effervescent preparations have found use as disclosed by Ashmead, et al. in United States Patent 4,725,427 and by Carnazzo in United States Patent 6,294,579.

Additionally, dietary products and nutritional supplements face new demands from the consumers depending on the group of consumers. Today, when people are travelling a lot, depending on the profession as well as being tourists, there is a need of low weight, stable and convenient products, which the consumer may bring along wherever they are. Additionally, the consumers are more conscious about what they look like and what they consume to improve their health and fitness. Therefore there is a need of developing new oil based compositions which full fill the requirement of being a stable, pleasant dietary product, which the consumers prefer to use.

SUMMARY OF THE INVENTION

The invention relates to a new composition for human consumption being substantially free from water. The composition being substantially free from water comprising a powder containing microencapsulated polyunsaturated long-chain esterified fatty acids (PUFA), wherein said PUFA content of said powder is from 5 to 50 %(w/w), said powder being homogenously distributed in an effervescent base, wherein said effervescent base is from 40 to 64 % (w/w) of said composition. Such a composition enables the possibility to protect the polyunsaturated long chain esterified fatty acid (PUFA) particles from oxidation and at the same time provides stability when the effervescent base is dissolved in water to form a homogenous dispersion of microencapsulated oil, which is easy to swallow. Further, the problems with most of the PUFA oils, in view of the unpalatable nature of a number of them, which have unpleasant odours, textures, flavours or other unpalatable properties have been eliminated as well as providing a defined high dose. Accordingly, the invented composition provides a combination of an immediate release and controlled release techniques, wherein the polyunsaturated long chain fatty acids, which are microencapsulated and do not release the oil until they have entered the gastrointestinal tract, become rapidly and homogenously dispersed in an aqueous solution. This being due to the fact that the effervescent base provides an environment in the liquid solution, where gas bubbles mixes the aqueous medium and the PUFA particles, making them move around in the water solution. Additionally, will the oil in the particles be released in a controlled way in the gastrointestinal tract, due to the fact that they are microencapsulated, either by erosion of the capsules in aqueous media or by enzymatic degradation.

Additionally, the invention relates to a process for the preparation of a composition comprising the steps of providing a powder containing microencapsulated polyunsaturated long-chain esterified fatty acids (PUFA), wherein said PUFA content of said powder is from 5 to 50 (w/w), providing at least one acid and at least one base forming an effervescent base, mixing said powder in said effervescent base and obtaining a composition, being substantially free from water, wherein said powder is homogenously distributed in said effervescent base and said composition having from 40 to 64 (w/w) % of said effervescent base.

Accordingly the invention relates to a tablet and/or granulate comprising the composition mentioned above and a process to produce such tablets or granules.

Finally, the invention relates to a dose unit, comprising said composition(s) or tablet(s) as well as containers comprising said dose unit.

Such an effervescent composition give rise to certain advantages, such as the possibility to incorporate large amounts of the active ingredients, such as an oil which do not need to be swallowed as a tablet. This is an advantage in connection to the high concentrated dose, which if consumed as it is would give rise to unpleasant taste and discomfort for the consumer. An effervescent can easily be dissolved in a liquid and then consumed without any unpleasant taste or discomfort. Additionally, by concentrating the dose into a small concentrated part the product itself will be easy to bring along and store. Alternatively, another convenient way is to use a two portion packaging, one of effervescent composition according to the invention and one of a liquid food, supplied to the consumer as one unit.

The composition is produced in a consumer friendly form, i.e, being practical since it is light to carry along and especially well suited for high consumers, travellers and for many senior citizens and children, it is easier to drink a glass of liquid than to swallow a capsule. The invention also makes it possible to administer relatively large doses, such as by using granules (without any practical problems).

DETAILED DESCRIPTION OF THE INVENTION

The invention relates to a composition being substantially free from water comprising a powder containing microencapsulated polyunsaturated long-chain esterified fatty acids (PUFA), wherein said PUFA content of said powder is from 5 to 50 %(w/w), said powder being homogenously distributed in an effervescent base, wherein said effervescent base is from 40 to 64 % (w/w) of said composition. The PUFA may be in an amount of from about 10 to about 40 %, such as from about 10-30 %, 15-20 %, 15-35 % or 20-30 %, (all values being w/w) or in an amount of about 10, 15, 20, 25, 30, 35, 40, 45 or 50 % Additionally, the effervescent base is in an amount from 40 to 64 (all values being w/w). The PUFA may be any PUFA such as omega-3 and/or omega-6 oils, such as oils having a high content of these PUFA's. Such a composition enables the possibility to keep PUFA which normally would oxidise in an non-oxidised form and thereby eliminate the unpleasant odours, textures, flavours which normally occur when oils are being oxidised, such as PUFA oils. Additionally the stability is increased since the PUFA do not oxidise and thereby the dose of the oils is controlled and easily regulated. Accordingly the composition can easily be dissolved and dispersed in a liquid, such as water or other liquid foods in a homogenous manner due to the unique combination of PUFA and a specific effervescent part. The effervescent base, upon contact with water, provides gas bubbles, which will move the particles containing PUFA around in the water. This means that the PUFA will remain homogenously dispersed in the liquid solution even though the particles have a density different from that of the water. Accordingly, the microencapsulation of the PUFA enables the possibility to get a release of the PUFA in the gastrointestinal tract, thereby minimising problems with unpleasant taste and burping.

The composition may comprise different kinds of PUFA as well as the PUFA's may be microencapsulated in different ways providing a mixture of different microencapsulated PuFA's as well as a mixture of different PUFA's.

*The microencapsulated oil part*

The PUFA may be obtained from vegetable, algae, microorganism or animal sources, such as a fish long chain polyunsaturated esterified fatty acid.

Microencapsulating of food and food components is a well-known technique for a person skilled in the art, which offer great value to manufacturers because they offer enhanced stability while delivering a desired fatty acid profile in a form that is easy to handle. The microencapsulating technique may use polysaccharides such as cellulose and starch, modified or unmodified, protein containing microcapsules or microencapsulated techniques such as spray-drying of emulsions as disclosed by Kolanowski et. Al., Int J Food Sci Nutr. 2004 Jun;55(4):333-43 and Hogan in J Microencapsul. 2003 Sep-Oct;20(5):675-88. Microencapsulated oil-based materials are produced by several companies, such as the Dutch company Kievit, Meppel, The Netherlands. An omega oil cyclodextrin powder is on the market under the brand name OmegaDry, by Wacker-Chemie GmbH Stuttgart Germany. Nu-Mega (Brisbane , Queensland, Australia) produces a tuna oil in a stable dry powder form and National Starch Food Innovation has introduced in the United States an encapsulated long-chain omega-3 fatty acid in powder form under the name NOVO-MEGA™ which contains an fish oil from Omega Protein Corporation (Houston Texas USA).

Microencapsulation may be at the molecular level, such as with inclusion within a cyclodextrin molecule, spray drying, coacervation and carrageenan entrapment. Examples of microencapsulation techniques include; emulsions of menhaden oil and sodium caseinate (NaCas) incorporating carbohydrates of varying dextrose equivalence (DE) spray-dried to yield encapsulated fish oil powders. Example of a microencapsulation technique is the MicroMAX® system, a microencapsulation technology developed by Food Science Australia in collaboration with Clover Corporation, overcomes the problems of fishy flavour by enclosing the fish oil in microcapsules and extending the shelf life of the product, MicroMAX® has an oil loading capability of 50%.

**MEG-3TM Omega-3 Powder and Meg-3TM Omega-3 DHA Powder use a novel** form of micro-encapsulation, which creates an unsurpassed "no taste, no smell" form of powdered fish oil ingredient for inclusion in food products. (Ocean Nutrition Canada Ltd).

**Vana-sana DHA 18 ES** is a high stability fish oil powder, jointly developed by Kievit and Lipid Nutrition. Use of a special sugar alcohol, known to have radical scavenging activity increased stabilising properties of the powder matrix. Other examples are encapsulation of wheat germ oil and evening primrose oil using the chemical reaction between the water-soluble sodium alginate and the polyvalent cation, calcium, to form the water-insoluble alginate sodium alginate has been reported.

**Gamma - cyclodextrin inclusion complex** of cranberry seed oil called OmegaDry® Cranberry is available from Wacker Specialties. OmegaDry Cranberry combines the nutritional properties of cranberry seed oil with the functional benefits of microencapsulation on the molecular level. It contains 45% cranberry seed oil.

The fatty acids according to the invention may be selected from the group of polyunsaturated fatty acids, such as linoleic acids, linolenic acids, eicosanoids fatty acids, omega-3 fatty acids and omega-6 fatty acids.

Examples of different polyunsaturated fatty acids (PUFA) and classes of PUFA are listed below:

- Linoleic Acids including conjugated ones and synthetic versions
- Linolenic Acids, such as Alpha-Linolenic Acid (ALA) and Gamma-Linolenic Acid (GLA)
- Eicosanoids, such as Eicosapentaenoic Acid (EPA) and Arachidonic Acid (ARA)
- Fatty Acids, Omega-3, such as Eicosapentaenoic Acid (EPA), Docosahexaenoic Acids (DHA) and Alpha-Linolenic Acid (ALA)
- Fatty Acids, Omega-6, such as Linoleic Acids and gamma-Linolenic Acid (GLA)

The PUFA in the composition may be selected from the group consisting of seal oil, flaxseed oil, evening primrose oil, algae oil, borage oil, hemp seed oil, perilla oil, blackcurrant seed oil, vitamin E, rice bran oil, cranberry oil, cod liver oil, tuna oil, anchovy oil, horse mackerel oil, sardine oil, menhaden oil, pilchards oil, corn oil, grape-seed oil, wheat germ oil, shark liver oil and olive oil. These oils are excellent sources for certain components.

Flaxseed oil, which is a source of alpha-linolenic acid (ALA) and evening primrose oil, which is a source of gamma-linolenic acid (GLA). Algae oil is rich in DHA. (e.g. microalgae-derived DHA oil, Martek Biosciences), borage oil being a source of gamma-linolenic acid (GLA), hemp seed oil being a source of alpha-linolenic acid (ALA) and GLA, perilla oil being a source of alpha-linolenic acid (ALA) and blackcurrant seed oil being a source gamma-linolenic acid (GLA), alpha-linolenic acid (ALA) and linoleic acid.

Further examples of other oils with health benefits are as follows; vegetable oils such as canola, sunflower, safflower, palm oil and wheat germ oils being rich in vitamin E, rice bran oil which contains three different kinds of natural antioxidants - - namely tocopherol, tocotrienol, and oryzanol, cranberry oil which contains tocopherols, tocotrienols, and phytosterols, cod liver oil being a one source of both vitamins A and D, flaxseed oil which contains varying amounts of lignan, corn oil which contains a natural mixture of both free and esterified phytosterols, grape-seed oil being rich in vitamin E and proanthrocyanidins, wheat germ oil containing octa-cosanol and shark liver oil and olive oil which contain squalene, a 30-carbon iso-prenoid. The different oils and oil sources mentioned above can be processed in different ways to increase the content of for example PUFA, or optimise the content of lipophilic bioactive compounds. "Structured lipids" (SL) are glycerides of fatty acids that have been modified to change the fatty acid composition and/or their positional distribution in the glycerol backbone by chemically and/or enzymatically catalyzed reactions and/or genetic engineering. More specifically, SLs are modified lipids with improved nutritional or functional properties. Eicosapentaenoic acid, 20: 5n-3 (EPA), and docosahexaenoic acid, 22:6n-3 (DHA), found in fish oil, are examples of n-3 polyunsaturated fatty acids (PUFAs) of interest in SL production.

Additionally, the composition may comprises one additive selected from the group comprising excipients, lubricants, preservatives, emulgators, pH adjusters, filler, surfactants, flavours, including natural or synthetic one, colours, vitamins, sweeteners, nutritional additives antioxidants, bacteria and mixtures thereof.

Examples of processes are transformations of lipids e.g. hydrolysis, esterification, and reesterification can increase the content of PUFA. Biochemical transformations have been review by Neklyudov et al 2002 "Biochemical Processing of Fats and Oils As a Means of Obtaining Lipid Products with Improved Biological and Physicochemical Properties: Applied Biochemistry and Microbiology 38: 399-409), in which expose of cold pressed oils to very mild process conditions such as, mechanical procedures without the application of heat. Cold pressed, nonrefined evening primrose oil (EPO) was recently found to contain lipophilic triterpenoidal esters with radical scavenging and anti-inflammatory properties. Cold pressed seed (e.g raspberry, black raspberry and boysenberry) oils have relatively high antioxidant activity. Supercritical Fluid Technology is powerful tool for the food and nutritional industry as for instance disclosed by Dunford et al. in United States Patent 6,677,469 discloses the use of a supercritical fluid fractionation process for phytosterol ester enrichment in vegetable oils. Molecular distillation is a refinement process for the concentration and purification of PUFA like EPA and DHA. As in any distillation process, it is based on molecular weight fractionation. Low molecular esters, such as ethanol and methanol esters are more easily distilled than the glyceride esters of fatty acids. However, other techniques may also be used as well as mixture of the above, mentioned techniques.

*The effervescent part*

Effervescence is the reaction (in water) of acids and bases producing carbon dioxide. The proportion of acids may be varied, as long as the bicarbonate is completely neutralised.

Examples of acids used in this reaction are citric acid, tartaric acid, malic acid, fumaric acid, adipic acid, acid citrates, succinic acid and mixtures thereof. Citric acid is the most commonly used, and it imparts a citrus-like taste to the product. Examples of bases used in the effervescent reaction are sodium carbonate, potassium carbonate, sodium bicarbonate, potassium bicarbonate, calcium bicarbonate, magnesium carbonate, sodium glycocarbonate, carboxylysine and mixtures thereof. Sodium bicarbonate is very common in effervescent formulas.

The above mentioned composition may comprise at least one additive selected from the group comprising excipients, lubricants, emulgators, fillers, surfactants (e.g., polysorbate 80 and sodium lauryl sulfate), flavours and colours, including natural or synthetic ones, vitamins, sweeteners (acesulfame potassium, sodium saccharin, aspartame, and surcalose), nutritional additives (e.g antioxidants), and mixtures thereof.

Substances giving taste, colour or antioxidative properties to the composition can be plant polyphenols (Cheynier V. Am J Clin Nutr.2005; 81: 223-229) coming from natural sources such as blueberries, cranberries, grapes and tea leaves.

The composition may include one or more excipient, such as a pharmaceutical excipient or a food additive. Examples of suitable excipients include, e.g., starches, natural gums, cellulose gums, microcrystalline cellulose, methylcellulose, cellulose ethers, sodium carboxymethylcellulose, ethylcellulose, gelatin, dextrose, lactose, sucrose, sorbitol, mannitol, polyethylene glycol, polyvinylpyrrolidone, pectins, alginates, polyacrylamides, polyvinyloxoazolidone, polyvinylalcohols and mixtures thereof.

Additionally the composition may contain various lubricants suitable for use in the composition including water dispersible, water soluble, water insoluble lubricants and combinations thereof. Examples of useful water soluble lubricants include sodium benzoate, polyethylene glycol, L-leucine, adipic acid, and combinations thereof.

The composition may also include water insoluble lubricants including, e.g., stearates (e.g., magnesium stearate, calcium stearate and zinc stearate), oils (e.g., mineral oil, hydrogenated and partially hydrogenated vegetable oils, and cotton seed oil) and combinations thereof. The effervescent part may also comprise vitamins, and minerals as disclosed in US 4,725,427 "Effervescent vitamin-mineral granule preparation". Effervescent energy tablet dissolving in any cold beverage (fruit juice, sports drinks, iced teas, sodas, etc.) to create an instant energy drink are commercially availed and such compositions may also be formulated according to the invention containing our invented composition.

The above, mentioned composition may be in the form of a tablet depending on the user and the use.

The invention also relates to a process for the preparation of a composition comprising the steps of; providing a powder containing microencapsulated polyunsaturated long-chain fatty acids (PUFA), mixing said powder in said effervescent base and obtaining a composition, wherein said microencapsulated PUFA is homogenously distributed and having at least 25 (w/w) % of said powder containing microencapsulated polyunsaturated long-chain fatty acids (PUFA) and from 40 to 64 (w/w) % of said effervescent base.

An effervescent product is an interesting dosage form offering some unique advantages compared with traditional tablets. The manufacturing process involves some critical steps that need to be addressed carefully during formulation and manufacturing, which is well known for a person skilled in the art. Production of effervescent products must occur in very low humidity areas. The best way to produce an effervescent product is in an environment where humidity is under strict control and to package it in a suitable moisture barrier.

Effervescent granules are sometimes prepared from a combination of citric and tartaric acid rather than from a single acid because the use of either acid alone causes difficulties. Because wet granulation will initiate the effervescent reaction, several alternatives have been established. Dry methods, such as slugging, direct compression and roller compaction are regularly used to produce solid dosage forms. Because the effervescent reaction is only started if the materials come into contact with water - and not if they come into contact with organic solvents - one possibility is to use such solvents as a granulation fluid.

Effervescent tablets are often mass-produced. The ideal amount of excipient is one that makes the tablet hard enough to handle, but soft enough to disintegrate (the harder the tablet, the slower the disintegration) and dry enough to be stable. The process of producing tablets, requires addition of pharmaceutical excipients well known to a person skilled in the art of powders like mixing, granulation and compression. It is common practice in tablet production to add a lubricant after granulation; the most commonly used substance is magnesium stearate. During effervescent production, substances such as magnesium stearate can generate a problem since they are insoluble in water and, consequently, a film will form on top of the water after the tablet has dissolved. Strategies to overcome this problem are the use of other lubricants that are soluble in water; for example, a mixture of spray dried L-leucine and polyethylene glycol. Alternatively, not using any lubricant has the advantage of avoiding the blending step, but the disadvantage of special requirements for the tablet press.

Said tablets and granules are consumer friendly in that they can be stored for period of time without degradation and/or oxidation of said oil-based material in airtight packages, such as containers. Aluminium, polyethylene and polypropylene, which have low water permeability, are used instead of standard polymer blister materials. If ten or more individual tablets are packed into one tube, very dry air can be added but when the tube is open to take out the first tablet, ambient moist air will enter and destroy the remaining effervescent tablets. To overcome this, silica gel or other drying agents are incorporated into most tube lids. The most common types of packaging are foil packets and tubes.

Further, the problems with most of the PUFA materials in relation to delivery of the these PUFA, in view of the unpalatable nature of a number of them, which have unpleasant odours, textures, flavours or other unpalatable properties have been reduced and/or eliminated. Additionally, the invented tablets and powders are easy to store and of low weight, which is important under certain circumstances such as when large quantities are consumed during shorter period of time. For example when sportsmen use the invented powders, granules or tablets. The invented tablets and powders may be packed into a container. Examples of container are tubes, bags, bottles and sachets, such as a daily dose package.

The invented compositions or tablets will preferably be used in a suspension such as being added to water prior to consumption. However, any kind of liquid may be used to dissolve the powders of tablets. Under certain circumstances it might be useful to dissolve the invention in a fruit drink, nutritional drink, milk or any kind of soft drink. In other embodiments of the invention is one serving of an aqueous drink packed in on container and the invented powders, granules and tablets in another container supplied to the customer as one product

Additionally, the invention relates to a process for the production of said composition comprising the steps of; providing a powder containing microencapsulated polyunsaturated long-chain esterified fatty acids (PUFA), wherein said PUFA content of said powder is from 5 to 50 (w/w), providing at least one acid and at least one base forming an effervescent base, mixing said powder in said effervescent base and obtaining a composition, being substantially free from water, wherein said powder is homogenously distributed in said effervescent base and said composition having from 40 to 64 (w/w) % of said effervescent base. The composition, being as defined above.

Accordingly the invention relates to a tablet and/or a granulate comprising the above defined composition. The granulates and tablet being produced by any technique which are well-known for a person skilled in the art. The invention also relates to a dose unit comprising the composition mentioned above, granulate or the tablet, also mentioned above. One or more of the dose units may be packaged in a, such as a tube, bag, bottle and sachets. In certain cases the container may further comprise at least one liquid, said dose unit and said liquid being separated from each other.

The invented composition is easily dissolved in a liquid and homogeneously dispersed in the liquid as well as stable.

The invention also relates to the use of said composition, tablet, granulate, dose unit or container as a dietary supplement.

The following examples are intended to illustrate, but not to limit, the invention in any manner, shape, or form, either explicitly or implicitly.

EXAMPLE 1

Effervescent tablet

An effervescent tablet (2100 mg) containing fish oil was prepared with the following composition:

| Ingredient | Amount per 100 g(g) | Amount per tablet (mg) | Function |
|---|---|---|---|
| Denomega-microencapsulated | 45.8 | 1000 (500 mg fish oil) | Microencapsulate cod-liver oil |

(continued)

| Ingredient | Amount per 100 g(g) | Amount per tablet (mg) | Function |
|---|---|---|---|
| Docusate Sodium | 0.19 | 4.1 | Surfactant |
| Acesulfame-K | 0.96 | 20.9 | Sweetener |
| Aspartame | 0.96 | 20.9 | Sweetener |
| Lemon lime flavouring | 2.88 | 62.8 | Flavouring agent |
| Citric acid | 22.9 | 500 | Effervescent agent |
| Sodium hydrogencarbonate | 26.3 | 573 | Effervescent agent |
| Target weight | 100.0 | 2180 | |

The microencapsulated fish oil was supplied by Denofa (Fredrikstad, Norge) all other components were of pharmacopoeia (European) quality.

The ingredients were mixed and a tablet was pressed which dissolved in 150 ml cold water in approximately 3 minutes and gave pH of 5.8. When the tablet is dissolved in a glass of milk, a pleasant tasting drink results.

EXAMPLE 2

Effervescent tablet

An effervescent tablet (2850 mg) was prepared with the following composition:

| Ingredient | Amount per tablet (mg) |
|---|---|
| Denomega-microencapsulated fish oil, (Denofa Fredistad Norway) | 500 |
| monosodium citrate | 1031 |
| Sodium bicarbonate | 809 |
| PEG 6000 | 100 |
| sorbitol | 400 |
| polysorbate 80 | 3 |
| sodium saccharinate | 8 |
| Target weight | 2851 |

The polysorbate was mixed with a part of sorbitol to give a homogeneous dry mixing in a tumbling mixer. After the above mixing, the remaining sorbitol and other components were blended together in the mixing device at 20-25.degrees C. and at about 20% RH. The resultant blend was made into a tablet and packaged in airtight containers.

EXAMPLE 3

Dispensing properties of Effervescent Omega-3 product.

Two batches of effervescent Omega-3 products were prepared in the following way, using the compositions listed below. Ingredient 2, 3 and 4 were mixed in a mortar and transferred to a glass beaker, to which the microencapsulated fish oil was added. The ingredients were first thoroughly mixed and then wetted with ethanol under further mixing. The damp powder was passed through a 0.8 mm sieve onto a glass plate and then dried in a microwave oven until solid granules were formed.

| Ingredient | Batch 1 Amount (g) | Batch 2 Amount (g) |
|---|---|---|
| 1. **Denomega-microencapsulated** fish oil, (Denofa Fredistad Norge) | 21 | 28 |

(continued)

| Ingredient | Batch 1 Amount (g) | Batch 2 Amount (g) |
|---|---|---|
| 2. Sodium bicarbonate | 11 | 11 |
| 3. Tartaric Acid | 6 | 6 |
| 4. Citric acid | 4 | 4 |

The prepared granules were compared with the pure Omega-3 powder in the following way. A spoon of granules (batch 1 and 2) and microencapsulated powder of Omega-3 oil were each added to a glass of grapefruit juice. The dispersing properties were studied visually. Both granulated effervescent products dispersed in less than 1 minute, while the microencapsulated fish oil powder lacked self dispersing properties and formed lumps of material.

EXAMPLE 4

Effervescent sachet containing cyclodextrin Omega-3 complex.

| Ingredient | Amount per batch (g) |
|---|---|
| 1.Cyclodextrin omega-3 inclusion complex | 93 |
| 2. Sodium Bicarbonate | 19,0 |
| 3.Mono sodium citrate | 24,2 |
| 4. Sorbitol, | 2.5 |
| 5.Lemon flavor (powder) | 0.6 |
| 6. Aspartame | 0.2 |
| Batch weight | 139.5 |

Ingredients were mixed in a tumbling mixer. A small aliquot of ethanol was added and the ingredients were mixed for a further 5 minutes before the wet product was granulated through a sieve (0.8 mm) and dried at 60°C under vacuum conditions. The resulting granules were filled into sachets, (target weight 6 g). The powder from one of the sachets was added to 200 ml water to give a sparkling, pleasant tasting liquid.

EXAMPLE 5

Composition of the effervescent formulation:

| Material | Amount (g) |
|---|---|
| Dry n-3 18:12 (BASF) | 96 |
| Acesulfam-K (Brøste) | 2 |
| Aspartam (NutraSweet) | 2 |
| Lemon aroma (Firmenich) | 9 |
| Citric acid, anhydrous (Roche) | 81 |
| Sodium hydrogen carbonate (Merck) | 110 |
| Target amount | 300 |

3 g (Φ 20 mm) tablets were produced from the above mixture. Tablets were introduced to 150 ml tap water and formed a homogeneous and good tasting liquid within 1-1.5 minutes. The high density particles with microencapsulated polyunsaturated oil are kept dispersed by attachment of gas bubbles formed from the effervescent composition. The liquid had the same appearance during the next two minutes i.e. particles rising and sinking in a bubbling aqueous solution. As a comparison, the powder of microencapsulated oil (1 g Dry n-3 18:12 from BASF AG) was easily dispersed in one glass of tap water (150 ml) with a spoon (5 sec) to form a dispersion, but the particles settled within 30 seconds to the bottom of the glass, with formation of a clear water phase above, making it impossible to swallow the designated dose of encapsulated oil with the water.

EXAMPLE 6

Determination of microencapsulation efficiency of microencapsulated powder:

The amount of extractable oil was determined by gently shaking 5 grams of microcapsulated PUFA with a total oil content of 48 % or 2,4 gram in 200 ml of petroleum ether for 10 minutes. The mixture was filtered through a Buchner funnel and the solvent was removed from a previously weighed flask on a rotary evaporator until a constant weight of the flaks was archived. Extractable oil, 0,05 gram, is the difference in weight between the clean flask and the weight of the flask after evaporation of petroleum ether,

The microencapsulation efficiency (MEE), 98 %, was calculated as follows.

$$MEE = (total\ oil - extractable\ oil) * 100/\ total\ oil$$

EXAMPLE 7

Sedimentation time for microencapsulated powder

2 g of microencapsulated PUFA powder, Dry n-3 18:12 from BASF, was added to 100 ml water in a measuring cylinder (100 ml), which after closure with a rubber stopper was vigorously shaken for 10 seconds.
The cylinder was placed on a table and after 1.5 minutes an 8 ml thick layer of particles had formed at the bottom of the cylinder.

**Claims**

1. A composition being substantially free from water comprising a powder containing microencapsulated polyunsaturated long-chain esterified fatty acids (PUFA), wherein said PUFA content of said powder is from 5 to 50 % (w/w), said powder being homogenously distributed in an effervescent base, wherein said effervescent base is from 40 to 64 % (w/w) of said composition.

2. The composition according to claim 1, wherein the PUFA content is from 10 to 40 % (w/w).

3. The composition according to claim 2, wherein the PUFA content is from 10 to 30 % (w/w).

4. The composition according to claim 3, wherein the PUFA content is from 15 to 20 % (w/w).

5. The composition according to any of preceding claims, wherein the PUFA content is 10, 15, 20, 25, 30, 35, 40, 45 or 50 % (w/w).

6. The composition according to any of preceding claims, wherein said PUFA is omega 3- and/or omega 6-oils.

7. The composition according to any of preceding claims, wherein said effervescent base comprises at least one acid selected from the group consisting of citric acid, tartaric acid, malic acid, fumaric acid, adipic acid, acid citrates, succinic acid and mixtures thereof and a base selected from the group consisting of sodium carbonate, potassium carbonate, sodium bicarbonate, potassium bicarbonate, calcium bicarbonate, magnesium carbonate, sodium glycocarbonate, carboxylysine and mixtures thereof.

8. The composition according to any of preceding claims, wherein said effervescent base comprises at least citric acid and sodium bicarbonate.

9. The composition according to any of preceding claims, wherein the powder comprises a mixture of different microencapsulated polyunsaturated long-chain fatty acids (PUFA).

10. The composition according to any of preceding claims, wherein the composition comprises at least one additive selected from the group comprising excipients, lubricants, preservatives, emulgators, pH adjusters, filler, surfactants, flavours, including natural or synthetic one, colours, vitamins, sweeteners, nutritional additives antioxidants, bacteria and mixtures thereof.

11. A process for the preparation of a composition comprising the steps of;

a) providing a powder containing microencapsulated polyunsaturated long chain esterified fatty acids (PUFA), wherein said PUFA content of said powder is from 5 to 50 (w/w),
b) providing at least one acid and at least one base forming an effervescent base,
c) mixing said powder in said effervescent base and
d) obtaining a composition, being substantially free from water, wherein said powder is homogenously distributed in said effervescent base and said composition having from 40 to 64 (w/w) % of said effervescent base.

12. The process according to claim 11, wherein the PUFA content is from 10 to 40 % (w/w) in said powder.

13. The process according to claim 12, wherein the PUFA content is from 10 to 30 % (w/w) in said powder.

14. The process according to claim 13, wherein the PUFA content is from 15 to 20 % (w/w) in said powder.

15. The process according to claims 11-14, wherein the PUFA content is 10, 15, 20, 25, 30, 35, 40, 45 or 50 % (w/w) in said powder.

16. The process according to claims 11-15, wherein said PUFA in said powder is omega 3- and/or omega 6-oils.

17. The process according to claims 11-16, wherein said effervescent base comprises at least one acid selected from the group consisting of citric acid, tartaric acid, malic acid, fumaric acid, adipic acid, acid citrates, succinic acid and mixtures thereof and a base selected from the group consisting of sodium carbonate, potassium carbonate, sodium bicarbonate, potassium bicarbonate, calcium bicarbonate, magnesium carbonate, sodium glycocarbonate, carboxylysine and mixtures thereof.

18. The process according to claims 17, wherein said effervescent base comprises at least citric acid and sodium bicarbonate.

19. A tablet and/or a granulate comprising a composition according to any of claims 1-10.

20. A dose unit comprising the composition according to claim 1-11 or the tablet or granulate according to claim 19.

21. A container comprising one or more dose units according to claim 20, such as a tube, bag, bottle and sachets.

22. The container according to claim 20, which further comprises at least one liquid, said dose unit and said liquid being separated from each other.

23. Use of the composition according to any of claims 1-11, the tablet or granulate according to claim 19, the dose unit according to claim 20 or the container according to claims 21-22 as a dietary supplement product.


**Patentansprüche**

1. Zusammensetzung, die im wesentlichen frei von Wasser ist und ein Pulver umfasst, das mikroverkapselte mehrfach ungesättigte langkettige veresterte Fettsäuren (PUFA) enthält, worin der PUFA-Gehalt des Pulvers von 5 bis 50 % (G/G) ist, das Pulver homogen in einem Brause-Basismaterial verteilt ist, worin das Brause-Basismaterial von 40 bis 64 % (G/G) der Zusammensetzung ausmacht.

**2.** Zusammensetzung gemäß Anspruch 1, worin der PUFA-Gehalt von 10 bis 40 % (G/G) ist.

**3.** Zusammensetzung gemäß Anspruch 2, worin der PUFA-Gehalt von 10 bis 30 % (G/G) ist.

**4.** Zusammensetzung gemäß Anspruch 3, worin der PUFA-Gehalt von 10 bis 20 % (G/G) ist.

**5.** Zusammensetzung gemäß irgendeinem der vorhergehenden Ansprüche, worin der PUFA-Gehalt 10, 15, 20, 25, 30, 35, 40, 45 oder 50 % (G/G) ist.

**6.** Zusammensetzung gemäß irgendeinem der vorhergehenden Ansprüche, worin die PUFA Omega-3- und/oder Omega-6-Öle sind.

**7.** Zusammensetzung gemäß irgendeinem der vorhergehenden Ansprüche, worin das Brause-Basismaterial mindestens eine Säure, ausgewählt aus der Gruppe bestehend aus Zitronensäure, Weinsäure, Äpfelsäure, Fumarsäure, Adipinsäure, sauren Zitraten, Bernsteinsäure und Mischungen daraus, sowie eine Base, ausgewählt aus der Gruppe bestehend aus Natriumcarbonat, Kaliumcarbonat, Natriumbicarbonat, Kaliumbicarbonat, Kalziumbicarbonat, Magnesiumcarbonat, Natriumglycocarbonat, Carboxylysin und Mischungen daraus, umfasst.

**8.** Zusammensetzung gemäß irgendeinem der vorhergehenden Ansprüche, worin das Brause-Basismaterial zumindest Zitronensäure und Natriumbicarbonat umfasst.

**9.** Zusammensetzung gemäß irgendeinem der vorhergehenden Ansprüche, worin das Pulver eine Mischung von verschiedenen mikroverkapselten mehrfach ungesättigten langkettigen Fettsäuren (PUFA) umfasst.

**10.** Zusammensetzung gemäß irgendeinem der vorhergehenden Ansprüche, worin die Zusammensetzung mindestens ein Additiv umfasst, das aus der Gruppe ausgewählt ist, die Hilfsstoffe, Gleitmittel, Konservierungsstoffe, Emulgatoren, pH-Regulatoren, Füllstoffe, Tenside, Geschmacksstoffe, einschließlich natürlicher oder synthetischer, Färbemittel, Vitamine, Süßmittel, Nährstoffadditive, Antioxidantien, Bakterien und Mischungen daraus umfasst.

**11.** Verfahren zur Herstellung einer Zusammensetzung, umfassend die Schritte:

a) Bereitstellen eines Pulvers, das mikroverkapselte mehrfach ungesättigte langkettige veresterte Fettsäuren (PUFA) enthält, worin der PUFA-Gehalt des Pulvers von 5 bis 50 % (G/G) ist,
b) Bereitstellen mindestens einer Säure und mindestens einer Base, die ein Brause-Basismaterial bilden,
c) Einmischen des Pulvers in das Brause-Basismaterial und
d) Erhalten einer Zusammensetzung, die im wesentlichen wasserfrei ist, worin das Pulver im Brause-Basismaterial homogen verteilt ist und die Zusammensetzung 40 bis 64 % (G/G) des Basismaterials aufweist

**12.** Verfahren gemäß Anspruch 11, worin der PUFA-Gehalt von 10 bis 40 % (G/G) ist.

**13.** Verfahren gemäß Anspruch 12, worin der PUFA-Gehalt von 10 bis 30 % (G/G) ist.

**14.** Verfahren gemäß Anspruch 13, worin der PUFA-Gehalt von 10 bis 20 % (G/G) ist.

**15.** Verfahren gemäß irgendeinem der vorhergehenden Ansprüche, worin der PUFA-Gehalt 10, 15, 20, 25, 30, 35, 40, 45 oder 50 % (G/G) ist.

**16.** Verfahren gemäß irgendeinem der vorhergehenden Ansprüche, worin die PUFA Omega-3- und/oder Omega-6-Öle sind.

**17.** Verfahren gemäß einem der Ansprüche 11 bis 16, worin das Brause-Basismaterial mindestens eine Säure, ausgewählt aus der Gruppe bestehend aus Zitronensäure, Weinsäure, Äpfelsäure, Fumarsäure, Adipinsäure, sauren Zitraten, Bernsteinsäure und Mischungen daraus, sowie eine Base, ausgewählt aus der Gruppe bestehend aus Natriumcarbonat, Kaliumcarbonat, Natriumbicarbonat, Kaliumbicarbonat, Kalziumbicarbonat, Magnesiumcarbonat, Natriumglycocarbonat, Carboxylysin und Mischungen daraus, umfasst.

**18.** Verfahren gemäß Anspruch 17, worin das Brause-Basismaterial zumindest Zitronensäure und Natriumbicarbonat umfasst.

**19.** Tablette und/oder Granulat, das eine Zusammensetzung gemäß irgendeinem der Ansprüche 1 bis 10 umfasst.

**20.** Dosiseinheit, die die Zusammensetzung gemäß Anspruch 1 bis 10 oder die Tablette oder das Granulat gemäß Anspruch 19 umfasst.

**21.** Behälter, der ein oder mehrere Dosiseinheiten gemäß Anspruch 20 umfasst, wie eine Röhre, Tasche und Beutel.

**22.** Behälter gemäß Anspruch 20, der weiterhin mindestens eine Flüssigkeit umfasst, worin die Dosiseinheit und die Flüssigkeit voneinander getrennt sind.

**23.** Verwendung der Zusammensetzung gemäß irgendeinem der Ansprüche 1 bis 10, der Tablette oder des Granulats gemäß Anspruch 19, der Dosiseinheit gemäß Anspruch 20 oder des Behälters gemäß Ansprüchen 21 bis 22 als Nahrungsergänzungsmittel.

**Revendications**

**1.** Composition sensiblement dépourvue d'eau comprenant une poudre contenant des acides gras estérifiés polyinsaturés à chaîne longue (PUFA) microencapsulés, dans laquelle la teneur en lesdits PUFA de ladite poudre est de 5 à 50 % (p/p), ladite poudre étant répartie de manière homogène dans une base effervescente, dans laquelle ladite base effervescente représente 40 à 64 % (p/p) de ladite composition.

**2.** Composition selon la revendication 1, dans laquelle la teneur en PUFA est de 10 à 40 % (p/p).

**3.** Composition selon la revendication 2, dans laquelle la teneur en PUFA est de 10 à 30 % (p/p).

**4.** Composition selon la revendication 3, dans laquelle la teneur en PUFA est de 15 à 20 % (p/p).

**5.** Composition selon l'une quelconque des revendications précédentes, dans laquelle la teneur en PUFA est de 10, 15, 20, 25, 30, 35, 40, 45 ou 50 % (p/p).

**6.** Composition selon l'une quelconque des revendications précédentes, dans laquelle lesdits PUFA sont des huiles oméga 3 et/ou oméga 6.

**7.** Composition selon l'une quelconque des revendications précédentes, dans laquelle ladite base effervescente comprend au moins un acide choisi dans le groupe constitué par l'acide citrique, l'acide tartrique, l'acide malique, l'acide fumarique, l'acide adipique, des citrates acides, l'acide succinique et les mélanges de ceux-ci et une base choisie dans le groupe constitué par le carbonate de sodium, le carbonate de potassium, le bicarbonate de sodium, le bicarbonate de potassium, le bicarbonate de calcium, le carbonate de magnésium, le glycocarbonate de sodium, la carboxylysine et les mélanges de ceux-ci.

**8.** Composition selon l'une quelconque des revendications précédentes, dans laquelle ladite base effervescente comprend au moins de l'acide citrique et du bicarbonate de sodium.

**9.** Composition selon l'une quelconque des revendications précédentes, dans laquelle la poudre comprend un mélange de différents acides gras polyinsaturés à chaîne longue (PUFA) microencapsulés.

**10.** Composition selon l'une quelconque des revendications précédentes, où la composition comprend au moins un additif choisi dans le groupe comprenant des excipients, des lubrifiants, des conservateurs, des émulsifiants, des agents ajustant le pH, des charges, des surfactants, des arômes, y compris naturels ou synthétiques, des colorants, des vitamines, des édulcorants, des antioxydants additifs nutritionnels, des bactéries et des mélanges de ceux-ci.

**11.** Procédé de préparation d'une composition comprenant les étapes suivantes :

a) la fourniture d'une poudre contenant des acides gras estérifiés polyinsaturés à chaîne longue (PUFA) microencapsulés, où la teneur en lesdits PUFA de ladite poudre est de 5 à 50 % (p/p),
b) la fourniture d'au moins un acide et d'au moins une base formant une base effervescente,
c) le mélange de ladite poudre dans ladite base effervescente et

d) l'obtention d'une composition, sensiblement dépourvue d'eau, dans laquelle ladite poudre est répartie de manière homogène dans ladite base effervescente et ladite composition comprenant de 40 à 64 % (p/p) de ladite base effervescente.

12. Procédé selon la revendication 11, dans lequel la teneur en PUFA est de 10 à 40 % (p/p) dans ladite poudre.

13. Procédé selon la revendication 12, dans lequel la teneur en PUFA est de 10 à 30 % (p/p) dans ladite poudre.

14. Procédé selon la revendication 13, dans lequel la teneur en PUFA est de 15 à 20 % (p/p) dans ladite poudre.

15. Procédé selon les revendications 11 à 14, dans lequel la teneur en PUFA est de 10, 15, 20, 25, 30, 35, 40, 45 ou 50 % (p/p) dans ladite poudre.

16. Procédé selon les revendications 11 à 15, dans lequel lesdits PUFA dans ladite poudre sont des huiles oméga 3 et/ou oméga 6.

17. Procédé selon les revendications 11 à 16, dans lequel ladite base effervescente comprend au moins un acide choisi dans le groupe constitué par l'acide citrique, l'acide tartrique, l'acide malique, l'acide fumarique, l'acide adipique, des citrates acides, l'acide succinique et des mélanges de ceux-ci et une base choisie dans le groupe constitué par le carbonate de sodium, le carbonate de potassium, le bicarbonate de sodium, le bicarbonate de potassium, le bicarbonate de calcium, le carbonate de magnésium, le glycocarbonate de sodium, la carboxylysine et des mélanges de ceux-ci.

18. Procédé selon la revendication 17, dans lequel ladite base effervescente comprend au moins de l'acide citrique et du bicarbonate de sodium.

19. Comprimé et/ou granulés comprenant une composition selon l'une quelconque des revendications 1 à 10.

20. Dose unitaire comprenant la composition selon les revendications 1 à 11 ou le comprimé ou les granulés selon la revendication 19.

21. Récipient comprenant une ou plusieurs doses unitaires selon la revendication 20, comme un tube, une poche, une bouteille et des sachets.

22. Récipient selon la revendication 20, qui comprend en outre au moins un liquide, ladite dose unitaire et ledit liquide étant séparés l'un de l'autre.

23. Utilisation de la composition selon l'une quelconque des revendications 1 à 11, du comprimé ou des granulés selon la revendication 19, de la dose unitaire selon la revendication 20 ou du récipient selon les revendications 21 et 22, en tant que complément alimentaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4895725 A
- EP 266323 A
- US 4725427 A
- US 6294579 B
- US 6677469 B

**Non-patent literature cited in the description**

- **KOLANOWSKI.** *Int J Food Sci Nutr.,* June 2004, vol. 55 (4), 333-43
- **HOGAN.** *J Microencapsul.,* September 2003, vol. 20 (5), 675-88
- **NEKLYUDOV et al.** Biochemical Processing of Fats and Oils As a Means of Obtaining Lipid Products with Improved Biological and Physicochemical Properties. *Applied Biochemistry and Microbiology,* 2002, vol. 38, 399-409
- **CHEYNIER V.** *Am J Clin Nutr.,* 2005, vol. 81, 223-229